# EUROPEAN PATENT SPECIFICATION

(11) **EP 0 287 900 B1**
(45) Date of publication and mention of the grant of the patent: **04.08.1993**
(21) Application number: 88105532.1
(22) Date of filing: 07.04.1988
(51) Int. Cl.: G01N 35/04, B01L 3/00

(54) **Locking rack and disposable sample cartridge**
Verschliess-Einschubbehälter und zugehöriger Wegwerf-Probenträger
Support de fixation et cartouche pour échantillons jetable

(30) Priority: 22.04.1987 US 41189
(43) Date of publication of application: 26.10.1988
(73) Proprietor: ABBOTT LABORATORIES, Abbott Park, Illinois 60064-3500 (US)
(72) Inventor: Grandone, Cass J., Lake Forest Illinois 60045 (US)
(74) Representative: Modiano, Guido, Dr.-Ing.

(56) References cited:
- EP-A- 0 192 968
- EP-A- 0 217 000
- EP-A- 0 217 403
- EP-A- 0 252 632
- WO-A-85/05451
- US-A- 3 441 383
- US-A- 4 235 840

## Description

The present invention relates to an apparatus for the accurate positioning of assays with respect to reading apparatuses. More specifically, the present invention relates to a carousel on which a plurality of disposable cartridges containing solid-phase assays are expediently positioned with minimal effort to a high degree of accuracy with respect to an optical reading apparatus through a series of locating nubs and tabs.

Solid-phase procedures and apparatuses for performing immunoassays in general and enzyme immunoassays in particular are generally well known in the art. These immunoassays can be performed on biological samples such as blood, spinal fluid, urine, serum, and plasma, to name a few. One particularly cost effective apparatus which has been developed and adapted for use in conjunction with solid-phase procedures to perform a variety of assays (such as sandwich and competitive assays) is a disposable assay cartridge having a plurality of wells, with at least one reaction well. The reaction well generally contains a fibrous matrix positioned across its entrance and an absorbent material positioned below the fibrous matrix. Microparticles, contained in or introduced into the fibrous matrix, react with a sample and an analyte-containing reagent which have been added to the reaction well to form an immobilized complex on the matrix. The excess sample and reagent are washed through the matrix and captured in the absorbent material below.

The resulting assay may be read using known optical detection apparatuses. For example, using conventional solid-phase procedures, the analyte can be labelled or tagged with a fluorophor which, when excited by light of a known wavelength, fluoresces and emits light at a second known wavelength. The intensity of the emitted light is indicative of the concentration of the analyte in the biological sample. A conventional fluorometer is suitable for illuminating the fibrous matrix with a beam of light having the appropriate excitation wavelength. The fluorometer also detects the intensity of the light at the emission wavelength. Assays using this type of solid-phase technology have been found to provide a high degree of sensitivity.

Disposable assay cartridges such as those described above are particularly well suited for use in automated assay preparation and reading equipment. Due to the high degree of sensitivity of assays using the fibrous matrix technology, it is imperative in such automated equipment that the assay-containing reaction well of each and every cartridge be positioned with a high degree of accuracy in each of three dimensions with respect to the optical reading apparatus in order to ensure that the readings have a repeatable high degree of accuracy.

The assays must not only be precisely positioned, they must be effortlessly and transparently positioned by even an unskilled operator with the same high degree of accuracy, in order to reduce the time and cost of each assay. That is, when the assays can be performed and read in a mass production-type manner, the unit cost for such assays decreases. In addition, the assay results can be made available more quickly.

A variety of automated assay equipment is known in the art. Such equipment typically includes apparatuses for moving various types of assay containers between certain assay preparation stations. These known apparatuses also position the prepared assays in proximity to various optical equipment for reading.

However, known moving apparatuses employed in such equipment are not suitable for use with disposable cartridge type assay containers of the previously described type. In addition, although some such apparatuses have in the past included locking means for retaining individual assay containers, such apparatuses have locked means to provide the precise assay positioning necessary to obtain highly accurate and repeatable optical readings.

For instance, in some known equipment the assays are placed in individual cuvettes or test tubes in a linear arrangement for movement in conveyor belt fashion past various preparation stations and ultimately to a reading station. Yet, these conveyor belts are difficult to accurately position, both in the path of movement as well as perpendicular to that path. In addition, when arranged in this fashion, the assays are sometimes jostled, thus producing elevational variations which result in inaccurate readings.

Known from EP-A-0,192,968 is a sealed reagent container structure having a body portion and a port-defining neck structure, which is upstanding from an upper wall of the body portion and located asymmetrically so that it is close to an end wall of the body portion. The body portion also has a bottom wall provided with a coupling structure for interengagement with a transport mechanism so that a series of containers can be moved past a reagent station for transferring material from the reagent container to analysis cuvettes.

Also known from EP-A-0,252,632, which designates DE,ES,FR,GB,IT, and NL and which constitutes prior art within the meaning of Art. 54(3) EPC, is a reagent cartridge having a plurality of storage compartments each having connecting members formed integrally therewith for vertically-stacked connection to an adjacent compartment. Wall members of the compartments are separated to prevent migration of contents between compartments, and a neck having a collar is provided to accommodate a transfer probe.

Known from EP-A-0,217,000 is an apparatus as defined in the precharacterizing part of claim 1.

Assays have been arranged in individual cuvettes or test tubes on a rotatable carousel rack. The carousel is circularly indexed to sequentially position the assays at various preparation stations and ultimately at a reading station containing an optical reading apparatus, such as a fluorometer. In this instance, the carousel has three potential positioning inaccuracies. The cuvette may be improperly positioned 1) radially with respect to the center of the carousel, 2) vertically with respect to the optical axis, and 3) laterally with respect to the radial axis in which the cuvette is held in the carousel. In addition, all of the previously used apparatuses have failed to provide means for effortlessly and transparently loading, locking, and precisely positioning a plurality of test tubes, cuvettes or other assay containers on the carousel, as well as for releasing such containers after the assays have been read.

### SUMMARY OF THE INVENTION

According to one aspect of the present invention, the above-mentioned problems are overcome by an apparatus for holding assay and other biological sample-containing cartridges for use in a biological sample analyzer, as defined in the appended claims.

According to another aspect of the invention, the above-mentioned problems are overcome by a disposable cartridge as defined in the appended claims.

The present invention, therefore, ensures the highly accurate positioning of a plurality of assays for optical reading while simultaneously ensuring the expeditious, effortless, and transparent loading, locking, and precise positioning of the assays. In this way, the automated mass positioning of the assays compliments speed and accuracy of the assay results.

The device of the present invention is a rack apparatus, which is a circular carousel, into which are placed a plurality of disposable assay cartridges. The cartridges are placed in a plurality of openings arranged around a central core of the carousel and specially formed to hold the cartridges. All cartridges have individual alignment features that insure their accurate alignment within the openings of the rack. These alignment members consist of a locator nub on the outer edge of the cartridge adapted to fit within a locator notch on the outer circumference of the rack. In addition, the inner edge of the cartridge contains two locator tabs. These locator tabs fit under a locking device placed over both the central core of the rack and the inner edge of each cartridge. This locking mechanism enables an operator to effortlessly and transparently lock the cartridges into precise positions within the openings with a single rotational actuation of the mechanism.

A plurality of locking tangs on the locking mechanism are adapted to simultaneously contact and push against corresponding pairs of locator tabs on each disposable cartridge which in turn, push the locator nubs into the locator notches when the locking mechanism is actuated. The outer edge of each cartridge is also beveled and is urged into secure mating contact with a similarly beveled undercut lip on the outer circumference of the rack. The locator notch and undercut lip both serve to prevent the rocking of the cartridges. The locator nubs, notches, tabs, bevels and tangs all prevent motion in any of the directional degrees of freedom. With such accurate positioning, the precise location of each disposable cartridge on the carousel is guaranteed.

The rack containing the cartridges can be circularly indexed to accurately position each assay-containing cartridge relative to a reading station containing an optical reading apparatus. Because the reading positioning is highly accurate, the assay is, with regularity, properly positioned for reading at the reading station.

The device of the present invention also provides effortless, expedient, and safe disposal of the cartridges following the readings of the assays. The operator simply de-actuates the locking mechanism with a single rotational de-actuation and inverts the carousel to release the cartridges.

### DESCRIPTION OF THE DRAWINGS

Further aspects and benefits of this device will become apparent through observing the attached drawings and detailed description of this invention in which:
FIG. 1 is an exploded perspective view of the preferred embodiment of the present invention.
FIG. 2 is a plan view of a disposable cartridge suitable for use with the preferred embodiment of the present invention.
FIG. 3 is an elevation view in cross section of the cartridge of FIG. 2, shown placed within the rack of the present invention with its associated locking hub in place, across lines 3-3 of FIG. 2.
FIG. 4 is an elevation view in cross section showing a disposable cartridge of the present invention in an opening of the carousel rack and illustrating the locator nub of the cartridge, taken across lines 4-4 of FIG. 3.
FIG. 5 is an elevation view in cross section showing a disposable cartridge of the present invention in an opening of the carousel rack and illustrating the locator tabs of the cartridge, taken across lines 5-5 of FIG. 3.
FIG. 6 is an elevation view in partial cross-section of a preferred embodiment of the carousel rack of the present invention.
FIG. 7 is a close-up perspective view of the locator nub of a cartridge of the present invention shown in relationship with a corresponding locator notch of the carousel rack of the present invention.
FIG. 8 is a close-up perspective view of the locking hub of the present invention shown in relationship with the carousel rack of the present invention.
FIG. 9 is a close-up perspective view of the locator ramps of a cartridge of the present invention shown in mating relationship with the corresponding locator tangs of the locking hub of the present invention.
FIG. 10 is a cross-sectional view of the locator tabs of a cartridge shown in mating relationship with the corresponding locator tangs of the locking hub of the present invention and the carousel rack of the present invention, as taken across lines 10-10 of FIG. 9.

### DETAILED DESCRIPTION OF A PREFERRED EMBODIMENT OF THE PRESENT INVENTION

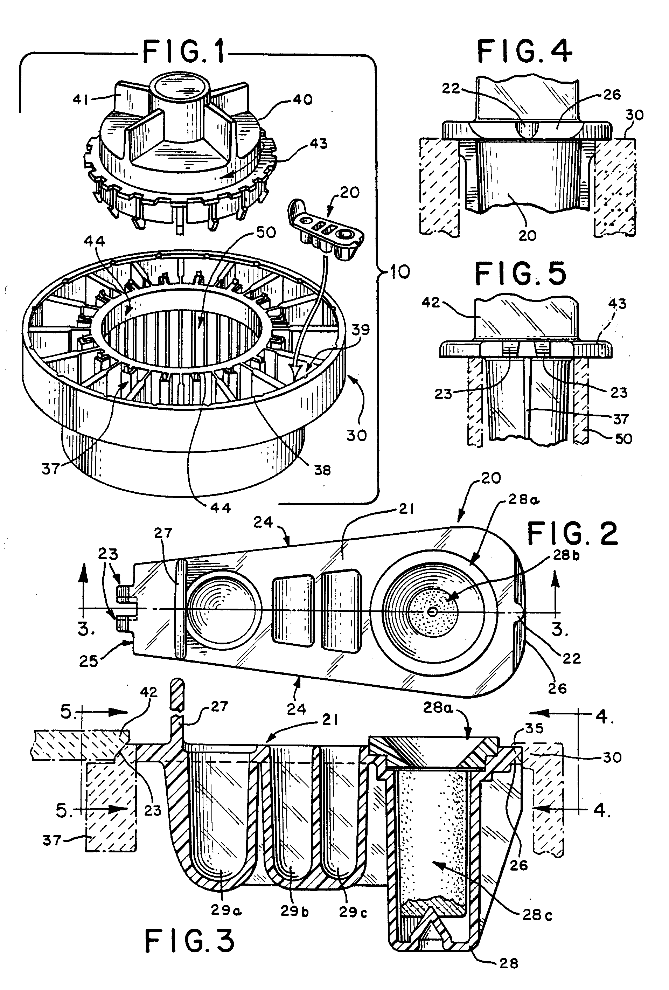

As seen from FIGS. 1-10, the preferred embodiment of the present invention comprises a carousel 10 having a circular rack 30 for holding a plurality of disposable assay cartridges 20, and a locking hub 40 which cooperates with the rack 30 to hold the cartridges 20 in place on the carousel 10. As seen in FIGS. 2 and 3, each of the cartridges 20 has a top surface 21 and a plurality of wells 28, 29a, 29b, and 29c. Well 28 is an assay reaction well and includes a funnel 28a, fibrous matrix 28b, and underlying absorbtive material 28c. The reaction well 28 is provided to contain an assay for reading under an optical reading apparatus, such as a fluorometer (not shown).

Each cartridge 20 has a pair of converging lateral sides 24 which correspond to converging sides of openings 39 located around the circumference of the rack 30. The cartridges 20 are preferably formed from injection molded ABS and SAN or equivalent plastics. Each of the lateral sides 24 is connected by an outer edge 26 and an inner edge 25 which correspond to outer and inner walls 38 and 44 respectively, of the openings 39 in the rack 30. Each of the cartridges 20 also contains a locator nub 22 centered on its outer edge 26 and a pair of parallel, substantially vertical beveled locator tabs 23 on its inner edge 25. The locator nubs 22 and locator tabs 23 are used to accurately position each cartridge laterally within an opening 39 in the rack 30. Each cartridge also has a finger tab 27 which allows an operator to easily and quickly place the cartridges 20 into the openings 39 of the rack 30. From the foregoing, it should be apparent that the geometries of the openings 39 and cartridges 20 preferable correspond in such a way that the cartridges 20 can be loaded into the openings 39 with only one orientation. This ensures that the cartridges 20 are always properly loaded for reading.

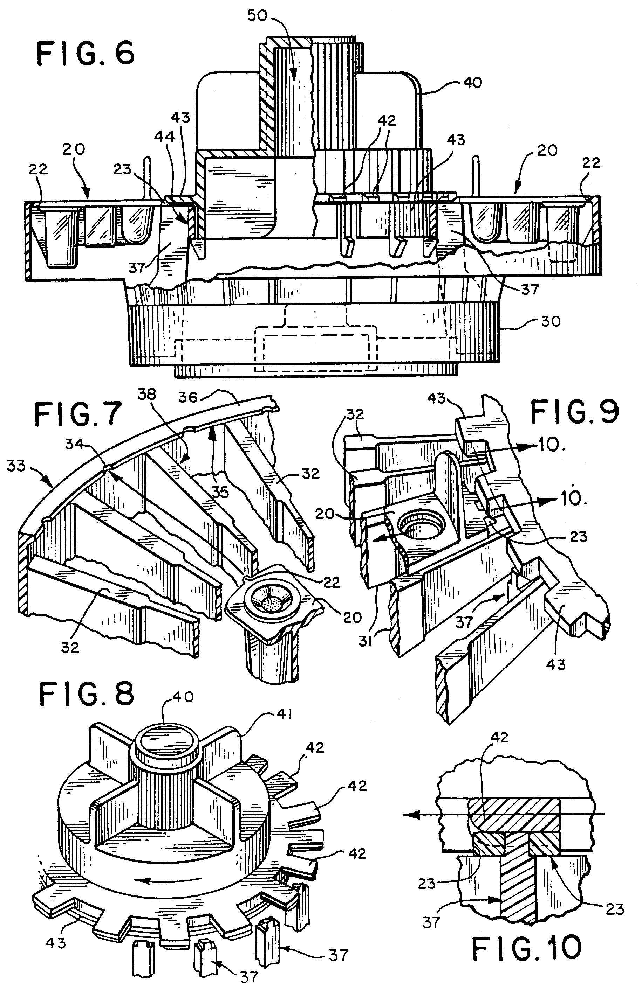

As seen in FIGS. 6, 7 and 9, the rack 30, which rotates about a central core 50 defined by a circular inner wall 44, contains a plurality of radially-projecting dividing walls 31 which, together with circular inner and outer walls 44 and 38, form the bottomless openings 39 into which each of the cartridges 20 fit. Each opening 39 isolates an individual cartridge 20 from every other cartridge. One advantage of such isolation is that each cartridge can be uniformly heated to a desired temperature during assay preparation by allowing warmed air to flow from beneath the carousel 10 into each of the openings 39. The rack 30 is preferably formed from an injection molded ABS or equivalent plastic. Each dividing wall 31 has a top reference plane 32 upon which sit the bottoms of the disposable cartridges 20. As seen in FIGS. 8-10, projecting radially from the circular inner wall 44 of each opening 39, there is a substantially vertical rail 37 which is adapted to fit between the beveled tabs 23 of cartridge 20. As seen in FIG. 7, on the outer circumference 33 of the rack there is a beveled undercut lip 35. Each undercut lip 35 has a plurality of locator notches 34, preferably centered in each opening 39. A locator nub 22 on each cartridge 20 is adapted to fit inside a corresponding locator notch 34 on the outer circumference 33 of the rack 30. The wedged outer edge 26 of the cartridge 20 also is adapted to fit against the undercut lip 35 on the outer circumference 33 of the rack 30. Thus, each cartridge 20 is restricted against movements in any plane of motion when mounted in the carousel rack 30.

As seen in FIGS. 1, 6 and 8, the locking hub 40 is comprised of a set of hand locking tabs 41 and an annular snapping cylinder 43 for mounting the locking hub 40 to be rotatable in the circular inner wall 44. As with the rack 30, the locking hub is preferably formed from an injection molded ABS or equivalent plastic. The hand locking tabs 41 allow locking hub 40 to be rotated in the circular inner wall 44 into locking position, as shown in FIG. 8. In addition, the locking hub 40 contains a plurality of hub locking tangs 42 which correspond to the number of openings 39 located around the circumference of the rack 30, as seen in FIGS. 8, 9, and 10. In the preferred embodiment, the hub locking tangs 42 are angularly sloped so that they fit against the beveled locator tabs 23 of the cartridges 20, when the cartridges are seated in each opening 39 and the locking hub 40 is rotated into locking position. When the locking hub 40 is rotated into locking position, the hub locking tangs 42 create a downward and outward radial force on the corresponding locator tabs 23 of the cartridges 20, so that the locator nubs 22 of the cartridges 20 are radially forced securely into the locator notches 34 of the rack 30. Also, a secure press fit is established between the beveled locator tabs 23 of each cartridge 20 and the hub locking tangs 42 of the locking hub 40 and between the wedged outer edge 26 of each cartridge 20 and the undercut lip 35 of the rack 30. Thus, each cartridge 20 is precisely positioned and secured against movement vertically, laterally, or radially within a corresponding opening 39.

As a result of the corresponding geometries of the cartridges 20 and openings 39, in operation, any number of cartridges can be effortlessly placed in each of the corresponding openings 39, aligned with the respective locking mechanisms 34 and 37 of the rack 30. When the locking hub 40 is manually rotated, the cartridge 20 in each opening 39 is individually precisely positioned and locked into place. This is all done with minimal initial positioning by the operator and accommodated with one effortless rotational locking motion. Thus, the function of precisely positioning the cartridges in each of three directions -- radially, laterally, and vertically -- is accomplished by the simple rotation of the locking hub and is completely transparent to the operator. Also, the cartridges 20 are expediently and safely removed from the rack 30 by simply rotating the locking hub 40 to unlock the cartridges 20, turning over the rack 30, and allowing the cartridges 20 to fall out effortlessly.

In the presently preferred embodiment, any number of disposable cartridge 20 are positioned, within a high degree of planar accuracy, in the rack 30. For instance, the cartridges 20 preferably have a nominal maximum width of approximately 2.093cm (0.824")_{,} which narrows linearly to approximately 1.074cm (0.436") over a distance of about 4.788cm (1.885"). The maximum lateral movement of the cartridge 20 in the opening 39 is preferably minimized to within approximately ±0.01016cm (±0.004") by the locator nubs 22 along with the locator tabs 23 on the ends of the disposable cartridges 20 and the corresponding locator notches 34 of the rack 30. The locator nubs 22 preferably have a nominal diameter of approximately 0.2032cm (0.080") with a tolerance of approximately ±0.00254cm (±.001"), and the locator notches 34 which receive the nubs each have a corresponding diameter. In addition, the vertical tolerance, that is, the elevational depth of the cartridges 20, is preferably held within a variation of less than ±0.01016cm (±0.004") due to the strict dimensional conformance made in the height of the disposable cartridges 20 when seated in the rack 30. The cartridges 20 are tightly held within the outer circumference 33 of the rack 30 by the corresponding locking tangs 42 on the locking mechanism 40 which create downward and radial forces that cause the disposable cartridges 20 to be locked into position against the outer edge of the rack 30. Both the locator tabs 23 and the hub locking tangs 42 on the locking hub preferably have a nominal mating width of approximately 0.01524cm (0.060") radially. The circumference of the undercut lip 35 is preferably concentric to within approximately ±0.00508cm (±.002") with respect to the inner wall 44. Consequently, the radial tolerance (the motion into and out of the center of the carousel 10) is preferably limited to approximately ±0.00508cm (±.002"). In this way, the accurate positioning necessary for accurate and repeatable optical reading of the assays in the reaction wells 26 of the cartridges 20 is ensured.

While the invention has been described in connection with the presently preferred embodiment, it should be immediately apparent to those skilled in the art that various changes and modifications to the structure, arrangement, portions, elements, materials, and components used in the practice of the invention which are particularly adapted for specific environments are possible. For example, it is understood that the specific geometries of the preferred locking and aligning mechanisms provided on the locking hub, the rack and the cartridges can be altered as long as the functional interrelationship of these elements provides the positioning and securing of the cartridges necessary to achieve the objectives of the invention. It is therefore intended that the foregoing description be regarded as illustrative rather than limiting, and it is understood that the following claims define the scope of this invention.

Where technical features mentioned in any claim are followed by reference signs, those reference signs have been included for the sole purpose of increasing the intelligibility of the claims and accordingly, such reference signs do not have any limiting effect on the scope of each element identified by way of example by such reference signs.

## Claims

1. Apparatus for holding assay and other biological sample-containing cartridges for use in a biological sample analyzer, comprising;
a plurality of cartridge means (20) for containing fluids, each of said cartridge means (20) having an inner edge (25) and an outer edge (26) connected by a pair of converging lateral sides (24);
a circular rack (30) having a circular inner wall (44) which defines a central core (50), a concentric outer wall, and a plurality of openings (39) defined between said inner and outer walls (44, 38) each for engaging one of said cartridge means (20) with said inner wall (44) adjacent to said inner edge (25) of said cartridge means (20), each of said cartridge means (20) containing two locator tabs (23) on said inner edge (25), and;
rotatable locking means (40) mounted on said central core (50) and having a plurality of tang means (42), operative to contact the locator tabs (23) of the cartridge means (20) when said locking means (40) is rotated,
characterized in that said openings (39) comprise laterally converging openings (39) defined by a plurality of angularly spaced radially projecting dividing walls (31) connected between said inner and outer walls (44, 38), each for receiving one of said cartridge means (20) with said outer edge (26) of said cartridge means (20) adjacent said outer wall (38) of said rack (30);
in that each said opening (39) contains a locator notch (34) formed in said outer wall (38) in alignment with a locator nub (22) formed on said outer edge (26) of each of said cartridge means (20), and a locator rail (37) formed on said inner wall (44) in alignment between said locator tabs (23);
and in that said tang means (42) are rotatable to simultaneously urge the locator nub (22) and locator tabs (23) of said cartridge means (20) into engagement with said locator notch (34) and said locator rail (37) of said opening (39) to lock said cartridge means (20) in a predetermined position in said opening (39).

2. Apparatus according to claim 1, characterized in that said locator rail (37) comprises a substantially vertical rail (37), whereby to restrict lateral movement of said cartridge means (39) in each of said openings (39).

3. Apparatus according to claim 1, characterized in that said outer edge (26) of said cartridge means (20) has an angled surface, and in that said outer wall (38) of said rack (30) includes a complementary angled surface (35), said angled surface being urged into secure mating contact with said complementary angled surface (35) when said locking means (40) is rotated.

4. Apparatus according to claims 1 and 3, characterized in that said angled surface is defined by a wedged outer edge (26) of said cartridge means (20).

5. Apparatus according to claims 1, 3 and 4, characterized in that said complementary angled surface (35) comprises an undercut lip (35) formed on the outer circumference (33) of said rack (30).

6. Apparatus according to claim 5, characterized in that said undercut lip (35) has a plurality of locator notches (34), each of said locator notches (34) being centered in one of said openings (39).

7. Apparatus according to claim 1, characterized in that said rotatable locking means (40) comprises a locking hub (40), and in that said tang means (42) comprise a plurality of hub-locking tangs (42) which are angularly sloped for exerting a downward and radial force on the corresponding locator tabs (23), whereby said locator nubs (22) of said cartridges (20) are radially forced securely into said locator notches (34) of said rack (30).

8. Apparatus according to claim 1, characterized in that said hub locking tangs (42) correspond in number to the number of openings (39) provided on said rack (30).

9. Apparatus according to claim 1, characterized in that said plurality of cartridge means comprises a plurality of disposable assay cartridges (20), each having a plurality of wells (28, 29a, 29b, 29c), one of said wells comprising a reaction well (28) including a fibrous solid-phase assay matrix (28b).

10. A disposable cartridge for use in the apparatus defined in one or more of claims 1-9 comprising a top surface (21) having an inner edge (25) and an outer edge (26) connected by a pair of converging lateral sides (24), a plurality of wells (28, 29a, 29b, 29c) formed in said top surface (21) for containing fluids, and two locator tabs (23) formed on said inner edge (25) for location adjacent said locking means (40), characterized in that it comprises a space defined between said locator tabs (23) for receiving and aligning with said locator rail (37), and a locator nub (22) formed on said outer edge (26) for alignment with said locator notch (34), for securing said cartridge in a predetermined radial, vertical and lateral position in said opening means (29).

11. A disposable cartridge according to claim 10, characterized in that it further comprises angled surface means (26) formed on said outer edge (26) and being locatable adjacent said complementary angled surface (35).

12. A disposable cartridge according to claim 10, characterized in that said plurality of wells (28, 29a, 29b, 29c) comprises at least one assay reaction well (28) including a fibrous matrix (28b).

13. A disposable cartridge according to claim 12, characterized in that said assay reaction well (28) further comprises absorptive material (28c) underlying said fibrous matrix (28b).

14. A disposable cartridge according to claim 12 or 13, characterized in that said assay reaction well (28) further comprises a funnel (28a).

15. A disposable cartridge according to claim 10 and 11, 12, 13 or 14, characterized in that said pair of converging lateral sides (24) are shaped to match said laterally converging openings (39) defined by said plurality of angularly spaced radially projecting dividing walls (31).

## Patentansprüche

1. Vorrichtung zur Aufnahme einer Probenanalyse und anderer Probenbehälterpatronen mit biologischen Proben zur Verwendung in einem biologischen Probenanalysator, bestehend aus:
einer Vielzahl von Patroneneinrichtungen (20) zur Aufnahme von Flüssigkeiten, wobei jede Patroneneinrichtung (20) einen inneren Rand (25) und einen äußeren Rand (26) aufweist, die durch ein Paar konvergierender, lateraler Seiten (24) verbunden sind;
einer ringförmigen Halterung (30) mit einer ringförmigen inneren Wand (44), die einen zentralen Kern (50) definiert, eine konzentrische äußere Wand, und eine Vielzahl von Öffnungen (39) zwischen den inneren und äußeren Wänden (44,38), jede zur Aufnahme der Patroneneinrichtung (20), wobei die innere Wand (44) am inneren Rand (25) der Patroneneinrichtung (20) anliegt, und jede Patroneneinrichtung (20) zwei Positionierungsvorsprünge (23) am inneren Rand (25) aufweist; und
einer drehbaren Verschlußvorrichtung (40), die am Kern (50) angebracht ist und eine Vielzahl von Zapfeneinrichtungen (42) enthalten, die in die Positionierungsvorsprünge (23) der Patroneneinrichtung (20) eingreifen, sobald die Verschlußvorrichtung (40) gedreht wird,
dadurch **gekennzeichnet,**
daß die Öffnungen (39) aus lateral konvergierenden Öffnungen (39) bestehen, die aus einer Vielzahl von winkelförmig angeordneten, in radialer Richtung ausgehenden Trennwänden (31) definiert sind, die mit den inneren und äußeren Wänden (44,38) verbunden sind, jede zur Aufnahme einer Patroneneinrichtung (20), wobei der äußere Rand (26) der Patroneneinrichtung (20) an der äußeren Wand (38) der Halterung (30) anliegt;
daß jede Öffnung (39) eine Stellkerbe (34) enthält, die in der äußeren Wand (38) gebildet ist und an einem am äußeren Rand (26) jeder Patroneneinrichtung (20) gebildeten Stellnippel (22) ausgerichtet ist, und eine Stellschiene (37), die an der inneren Wand (44) in Ausrichtung mit den Positionierungsvorsprüngen (23) gebildet ist;
und daß die Zapfeneinrichtungen (42) drehbar angeordnet sind, um gleichzeitig die Stellnippel (22) und die Positionierungsvorsprünge (23) der Patroneneinrichtung (20) in die Stellkerbe (34) und die Stellschiene (37) der Öffnungen (39) hineinzudrücken, um die Patroneneinrichtung (20) in einer vorherbestimmten Position in der Öffnung (39) zu verriegeln.

2. Vorrichtung nach Anspruch 1, dadurch gekennzeichnet, daß die Stellschiene (37) aus einer im wesentlichen vertikalen Schiene (37) besteht, wodurch eine seitliche Bewegung der Patroneneinrichtung (20) in jeder der Öffnungen (39) verhindert wird.

3. Vorrichtung nach Anspruch 1, dadurch gekennzeichnet, daß der äußere Rand (26) der Patroneneinrichtung (20) eine abgewinkelte Oberfläche enthält und daß die äußere Wand (38) der Halterung (30) eine komplementär gewinkelte Oberfläche (35) beinhaltet, wobei die abgewinkelte Oberfläche in sicherem Berührungskontakt mit der komplementär gewinkelten Fläche (35) gebracht wird, wenn die Verschlußvorrichtung (40) gedreht wird.

4. Vorrichtung nach den Ansprüchen 1 und 3, dadurch gekennzeichnet, daß die abgewinkelte Oberfläche aus dem eckigen äußeren Rand (26) der Patroneneinrichtung (20) besteht.

5. Vorrichtung nach den Ansprüchen 1, 3 und 4, dadurch gekennzeichnet, daß die komplementär gewinkelte Oberfläche (35) eine hinterschnittene Lippe (35) am äußeren Umfang (33) der Halterung (30) aufweist.

6. Vorrichtung nach Anspruch 5, dadurch gekennzeichnet, daß die hinterschnittene Lippe (35) eine Vielzahl von Stellkerben (34) besitzt, wobei jede Stellkerbe (34) in einer der Öffnungen (39) zentriert ist.

7. Vorrichtung nach Anspruch 1, dadurch gekennzeichnet, daß die drehbare Verschlußvorrichtung (40) eine Verschlußnabe (40) aufweist, und daß die Zapfeneinrichtungen (42) eine Vielzahl von Nabenarretierzapfen (42) enthalten, die abgeschrägt sind, um eine nach abwärts gerichtete und radiale Kraft auf die korrespondierenden Positionierungsvorsprünge (23) auszuüben, wobei die Stellnippel (22) der Patroneneinrichtung (20) in radialer Richtung sicher in die Stellkerben (34) der Halterung (30) gedrückt werden.

8. Vorrichtung nach Anspruch 1, dadurch gekennzeichnet, daß die Nabenarretierzapfen (42) in ihrer Anzahl der Anzahl der Öffnungen (39) auf der Halterung (30) entsprechen.

9. Vorrichtung nach Anspruch 1, dadurch gekennzeichnet, daß die Vielzahl der Patroneneinrichtungen eine Vielzahl von Wegwerfanalysenpatronen (20) enthalten, wobei jede mehrere Schächte (28,29a,29b,29c) enthält, von denen jeder Schacht eine Reaktionskammer (28) und eine fasrige solide Analysematrix (28b) enthält.

10. Wegwerfpatrone zum Gebrauch in der Vorrichtung nach einem oder mehreren der vorgehenden Ansprüche 1 bis 9, bestehend aus einer oberen Fläche (21) mit einem inneren Rand (25) und einem äußeren Rand (26), wobei beide Ränder durch ein Paar von konvergierenden Seitenflächen verbunden sind, mehreren Schächten (28,29a,29b,29c), die in der oberen Fläche (21) zur Aufnahme von Flüssigkeiten gebildet sind, und zwei Positionierungsvorsprüngen (23) am inneren Rand (25) für eine Positionierung angrenzend an die Verschlußvorrichtung (40), dadurch gekennzeichnet, daß diese einen Zwischenraum zwischen den Positionierungsvorsprüngen (23) enthält zur Aufnahme und Ausrichtung mit der Stellschiene (37), und einen Stellnippel (22), der am äußeren Rand (23) gebildet ist zur Ausrichtung mit der Stellkerbe (34), um die Patrone sicher in einer vorbestimmten radialen, senkrechten und lateralen Position in den Öffnungen (29) zu halten.

11. Wegwerfpatrone nach Anspruch 10, dadurch gekennzeichnet, daß diese weiterhin eine abgewinkelte Fläche (26) am äußeren Rand (26) enthält, die an die komplementäre abgewinkelte Fläche (35) anlegbar ist.

12. Wegwerfpatrone nach Anspruch 10, dadurch gekennzeichnet, daß die Vielzahl von Schächten (28,29a,29b, 29c) zumindestens einen Analysereaktionsschacht (28) mit einer Fasermatrix (28b) enthalten.

13. Wegwerfpatrone nach Anspruch 12, dadurch gekennzeichnet, daß der Analysereaktionsschacht (28) weiterhin saugfähiges Material (28c) unter der Fasermatrix (28b) enthält.

14. Wegwerfpatrone nach Anspruch 12 oder 13, dadurch gekennzeichnet, daß der Analysereaktionsschacht (28) weiterhin einen Trichter (28a) enthält.

15. Wegwerfpatrone nach den Ansprüchen 10 und 11, 12, 13 oder 14, dadurch gekennzeichnet, daß das Paar der konvergierenden Seiten (24) so gestaltet ist, daß es in die seitlich konvergierenden Öffnungen (39) paßt, die von der Vielzahl von abgewinkelt getrennten, radial reichenden Trennwänden (31) definiert sind.

## Revendications

1. Appareil destiné à contenir un dosage et d'autres cartouches contenant des échantillons biologiques en vue d'une utilisation dans un analyseur d'échantillons biologiques, comprenant :
une pluralité de moyens de cartouche (20) destinés à contenir des fluides, chacun desdits moyens de cartouche (20) comportant un bord interne (25) et un bord externe (26) connectés au moyen d'une paire de côtés latéraux convergents (24) ;
un magasin circulaire (30) comportant une paroi interne circulaire (44) gui définit une âme centrale (50), une paroi externe concentrique et une pluralité d'ouvertures (39) définies entre lesdites parois interne et externe (44, 38), chacune étant destinée à coopérer avec l'un desdits moyens de cartouche (20), ladite paroi interne (44) étant adjacente audit bord interne (25) desdits moyens de cartouche (20), chacun desdits moyens de cartouche (20) contenant deux pattes de positionnement (23) prévues sur ledit bord interne (25) ; et
un moyen de verrouillage tournant (40) monté sur ladite âme centrale (50) et comportant une pluralité de moyens de languette (42) opérant pour entrer en contact avec les pattes de positionnement (23) des moyens de cartouche (20) lorsque ledit moyen de verrouillage (40) est tourné,
caractérisé en ce que lesdites ouvertures (39) comprennent des ouvertures convergeant latéralement (39) définies par une pluralité de parois et de divisions se projetant radialement qui sont espacées angulairement (31) et qui sont connectées entre lesdites parois interne et externe (44, 38), chacune étant destinée à recevoir l'un desdits moyens de cartouche (20) tandis que ledit bord externe (26) desdits moyens de cartouche (20) est adjacent à ladite paroi externe (38) dudit magasin (30) ;
en ce que chaque dite ouverture (39) contient une encoche de positionnement (34) formée dans ladite paroi externe (38) de manière à être alignée avec un bouton de positionnement (22) formé sur ledit bord externe (26) de chacun desdits moyens de cartouche (20) et un rail de positionnement (37) formé sur ladite paroi interne (44) de manière à être aligné avec lesdites pattes de positionnement (23) ;
et en ce que lesdits moyens de languette (42) peuvent être tournés de manière à amener simultanément le bouton de positionnement (22) et les pattes de positionnement (23) desdits moyens de cartouche (20) en coopération avec ladite encoche de positionnement (34) et avec ledit rail de positionnement (37) de ladite ouverture (39) afin de verrouiller lesdits moyens de cartouche (20) dans une position prédéterminée dans ladite ouverture (39).

2. Appareil selon la revendication 1, caractérisé en ce que ledit rail de positionnement (37) comprend un rail sensiblement vertical (37) pour ainsi limiter tout déplacement latéral desdits moyens de cartouche (20) dans chacune desdites ouvertures (39).

3. Appareil selon la revendication 1, caractérisé en ce que ledit bord externe (26) desdits moyens de cartouche (20) présente une surface angulée et en ce que ladite paroi externe (38) dudit magasin (30) inclut une surface angulée complémentaire (35), ladite surface angulée étant poussée de manière à établir un contact de conjugaison ferme avec ladite surface angulée complémentaire (35) lorsque ledit moyen de verrouillage (40) est tourné.

4. Appareil selon les revendications 1 et 3, caractérisé en ce que ladite surface angulée est définie par un bord externe de fixation (26) desdits moyens de cartouche (20).

5. Appareil selon les revendications 1, 3 et 4, caractérisé en ce que ladite surface angulée complémentaire (35) comprend un rebord en dégagement (35) formé sur la circonférence externe (33) dudit magasin (30).

6. Appareil selon la revendication 5, caractérisé en ce que ledit rebord en dégagement (35) comporte une pluralité d'encoches de positionnement (34), chacune desdites encoches de positionnement (34) étant centrée dans l'une desdites ouvertures (39).

7. Appareil selon la revendication 1, caractérisé en ce que ledit moyen de verrouillage tournant (40) comprend un moyeu de verrouillage (40) et en ce que lesdits moyens de languette (42) comprennent une pluralité de languettes de verrouillage de moyeu (42) qui sont inclinées angulairement pour exercer une force radiale dirigée vers le bas sur les pattes de positionnement correspondantes (23) d'où il résulte que lesdits boutons de positionnement (22) desdites cartouches (20) sont forcées radialement fermement dans lesdites encoches de positionnement (34) dudit magasin (30).

8. Appareil selon la revendication 1, caractérisé en ce que lesdites languettes de verrouillage de moyeu (42) correspondent en nombre au nombre d'ouvertures (39) ménagées sur ledit magasin (30).

9. Appareil selon la revendication 1, caractérisé en ce que ladite pluralité de moyens de cartouche comprend une pluralité de cartouches de dosage prélevé jetables (20) dont chacune comporte une pluralité de puits (28, 29a, 29b, 29c), l'un desdits puits comprenant un puits de réaction (28) incluant une matrice de dosage fibreuse à phase solide (28b).

10. Cartouche jetable destinée à une utilisation dans l'appareil défini dans l'une ou plusieurs des revendications 1 à 9, comprenant une surface supérieure (21) comportant un bord interne (25) et un bord externe (26) connectés par une paire de côtés latéraux convergents (24), une pluralité de puits (28, 29a, 29b, 29c) formés sur ladite surface supérieure (21) pour contenir des fluides et deux pattes de positionnement (23) formées sur ledit bord interne (25) pour un positionnement adjacent audit moyen de verrouillage (40), caractérisée en ce qu'elle comprend un espace défini entre lesdites pattes de positionnement (23) destiné à recevoir et à s'aligner avec ledit rail de positionnement (37), et un bouton de positionnement (22) formé sur ledit bord externe (26) et prévu pour s'aligner avec ladite encoche de positionnement (34), pour fixer ladite cartouche en une position radiale, verticale et latérale prédéterminée dans ledit moyen d'ouverture (39).

11. Cartouche jetable selon la revendication 10, caractérisée en ce qu'elle comprend en outre un moyen de surface angulée (26) formée sur ledit bord externe (26), lequel moyen peut être positionné de manière à être adjacent à ladite surface angulée complémentaire (35).

12. Cartouche jetable selon la revendication 10, caractérisée en ce que ladite pluralité de puits (28, 29a, 29b, 29c) comprend au moins un puits de réaction de dosage (28) incluant une matrice fibreuse (28b).

13. Cartouche jetable selon la revendication 12, caractérisée en ce que ledit puits de réaction de dosage (28) comprend en outre un matériau absorbant (28c) s'étendant au-dessous de ladite matrice fibreuse (28b).

14. Cartouche jetable selon la revendication 12 ou 13, caractérisée en ce que ledit puits de réaction de dosage (28) comprend en outre un entonnoir (28a).

15. Cartouche jetable selon les revendications 10 et 11, 12, 13 ou 14, caractérisée en ce que ladite paire de côtés latéraux convergents (24) sont conformés de manière à correspondre auxdites ouvertures convergeant latéralement (39) définies par ladite pluralité de parois de division se projetant radialement et espacées angulairement (31).
